# EUROPÄISCHE PATENTSCHRIFT

(11) **EP 0 353 184 B1**
(45) Veröffentlichungstag und Bekanntmachung des Hinweises auf die Patenterteilung: **15.06.1994**
(21) Anmeldenummer: 89810523.4
(22) Anmeldetag: 11.07.1989
(51) Int. Cl.: C07D 487/04, G03G 5/06

(54) **Verfahren zur Herstellung aminierter Diketodi(het)aryl-pyrrolopyrrole und Verwendung derselben als photoleitfähige Substanzen**
Process for the preparation of aminated diketodi(het)aryl-pyrrolopyrroles and use of the same as photoconducting substances
Procédé pour la préparation de dicétodi(hét)aryl-pyrrolopyrroles aminés et l'application de ceux-ci comme composés photo-conducteurs

(30) Priorität: 20.07.1988 CH 2769/88
(43) Veröffentlichungstag der Anmeldung: 31.01.1990
(73) Patentinhaber: CIBA-GEIGY AG, 4002 Basel (CH)
(72) Erfinder: Rochat, Alain Claude, Dr., CH-1700 Fribourg (CH); Wallquist, Olof, Dr., CH-1723 Marly (CH); Iqbal, Abul, Dr., CH-1732 Arconciel (CH); Mizuguchi, Jin, Dr., CH-1700 Fribourg (CH)

(56) Entgegenhaltungen:
- EP-A- 0 061 426
- EP-A- 0 094 911
- EP-A- 0 187 620
- US-A- 3 484 487

## Beschreibung

Die vorliegende Erfindung betrifft ein Verfahren zum Aminieren von 1,4-Diketo-3,6-di(het)aryl-pyrrolo-[3,4-c]-pyrrolen. Die so erhaltenen Produkte können als photoleitfähige Substanzen verwendet werden.

1,4-Diketo-3,6-diaryl-pyrrolo-[3,4-c]-pyrrole, darunter auch Aminoarylderivate, werden beispielsweise in EP-A-61426 und EP-A 94911 offenbart. Solche Aminoarylderivate von 1,4-Diketo-pyrrolo-[3,4-c]-pyrrolen können nach den bisher bekannten Herstellungsmethoden, z.B. durch Umsetzung eines Bernsteinsäurediesters mit einem aminsubstituierten Benzonitril, nur schwer und mit geringer Ausbeute hergestellt werden.

Die US-A-3 484 487 beschreibt ein Verfahren zur Aminierung von Arylhalogeniden, wobei Arylhalogenide mit Aminen in Gegenwart eines polaren organischen Lösungsmittels umgesetzt werden. Dieses Verfahren wird bei hohen Drucken zwischen 34,5 und 103,5 bar (500-1500 psi) und bei Temperaturen zwischen 250 und 280°C durchgeführt.

Es ist nun gefunden worden, dass man aminierte Diketo-diaryl-pyrrolopyrrole überraschenderweise mit guten Ausbeuten erhalten kann, wenn man halogenierte Diketo-diaryl-pyrrolopyrrole mit sekundären Aminen in Gegenwart eines bestimmten wasserfreien, dipolaren, aprotischen Lösungsmittels und einer Base, bei einem Druck zwischen 1 und 3 bar, und bei Temperaturen zwischen 100 und 220°C umsetzt.

Aus EP-A-187620 ist bekannt, dass 1,4-Dithioketo-pyrrolo-[3,4-c]-pyrrole sich für den Einsatz als photoleitfähige Substanzen, insbesondere im nahen Infrarot-Bereich, eignen.

Es ist nun zusätzlich gefunden worden, dass aminierte 1,4-Diketo-3,6-di(het)aryl-pyrrolo-[3,4-c]-pyrrole, wie sie nach dem obenerwähnten neuen Verfahren erhältlich sind, überraschenderweise ebenfalls photoleitende Eigenschaften zeigen und zwar nicht im nahen Infrarot-, sondern schon im sichtbaren Wellenlängenbereich.

Die vorliegende Erfindung betrifft demnach ein Verfahren zur Herstellung von Verbindungen der Formel 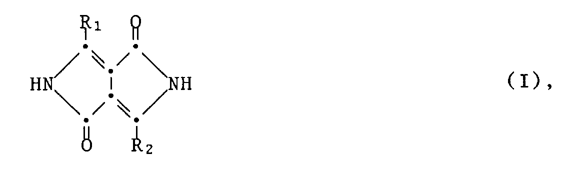
worin R₁ eine Gruppe 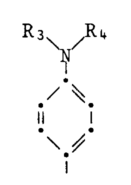
und R₂ eine Gruppe 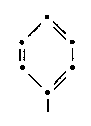
oder R₁ bedeuten, wobei R₃ und R₄ gleich sind und C₁-C₁₂-Alkyl, 2-Hydroxyethyl, 2-Mercaptoethyl, Cyclohexyl, Benzyl oder Phenylethyl bedeuten oder zusammen mit dem N-Atom, an das sie gebunden sind, Pyrrolidinyl, Piperidyl, Morpholinyl oder Thiomorpholinyl bilden,
durch Umsetzung eines Pyrrolopyrrols der Formel 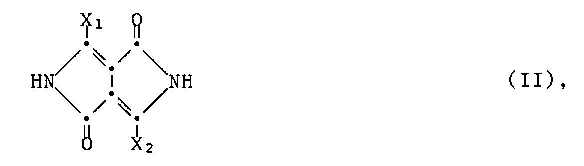
worin X₁ eine Gruppe 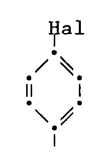
und X₂ eine Gruppe 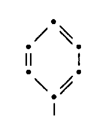
oder X₁ bedeuten, Hal Chlor oder Brom ist, mit einem sekundären Amin der Formel 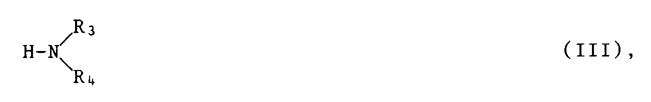
worin R₃ und R₄ die oben bereits angegebene Bedeutung haben, im Molverhältnis 1:1 oder, wenn X₂ die gleiche Bedeutung wie X₁ hat, im Molverhältnis 1:2, in Gegenwart eines wasserfreien, dipolaren, aprotischen Lösungsmittels ausgewählt aus der Gruppe bestehend aus Carbonsäureamiden, Lactamen, Harnstoffderivaten, Sulfonen und Nitrobenzol und eines als Base wirkenden, 0,1 bis 15 Mol betragenden Ueberschusses an Amin der Formel III bei Temperaturen zwischen 100 und 220°C und einem Druck zwischen 1 und 3 bar.

Beispiele von C₁-C₁₂-Alkylgruppen sind Methyl, Ethyl, n-Propyl, Isopropyl, n-Butyl, sec.-Butyl, tert.-Butyl, n-Pentyl, tert.-Amyl, n-Hexyl, 1,1,3,3-Tetramethylbutyl, n-Heptyl, n-Octyl, Nonyl, Decyl, Undecyl und Dodecyl.

Besonders bevorzugt wird das erfindungsgemässe Verfahren gemäss der oben dargelegten Bevorzugung, das dadurch gekennzeichnet ist, dass in den Formeln I, II und III R₁ und R₂ und demnach auch X₁ und X₂ gleich sind, R₃ und R₄ C₁-C₄-Alkyl, 2-Hydroxyethyl, 2-Mercaptoethyl bedeuten oder zusammen mit dem N-Atom, an das sie gebunden sind, Pyrrolidinyl, Piperidyl, Morpholinyl oder Thiomorpholinyl bilden, Hal Chlor oder Brom bedeuten, und dass die Umsetzung bei Temperaturen zwischen 150 und 200°C durchgeführt wird.

Ganz besonders bevorzugt wird das erfindungsgemässe Verfahren zur Herstellung von Verbindungen der Formel I, worin R₁ und R₂ gleich sind und eine der Gruppen 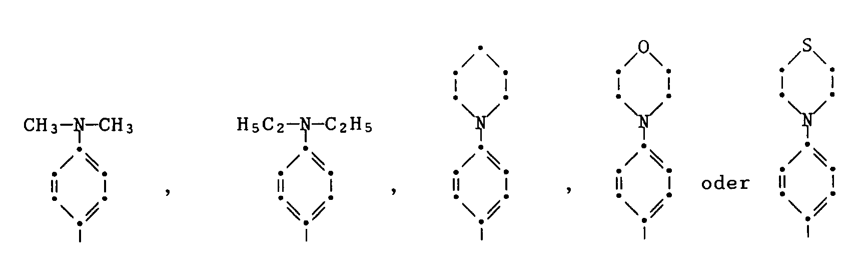
bedeuten.

Als Lösungsmittel werden Dimethylformamid, Dimethylacetamid, N-Methylpyrrolidon, N,N'-Dimethylethylenharnstoff und N,N'-Dimethylpropylenharnstoff bevorzugt.

Die Verbindungen der Formel I können, wie bereits aus den US-Patentschriften bekannt, als Pigmente eingesetzt werden. Sie können aber auch als Ausgangsprodukte für die Herstellung von Dithioketopyrrolo-[3,4-c]-pyrrolen gemäss der US-A-4 632 893 verwendet werden.

Von besonderem Interesse sind die Verbindungen der Formel I als photoleitfähige Substanzen in elektrophotographischen Photorezeptoren. Solche Photorezeptoren bestehen aus einer leitfähigen Unterlage und einem Photoleiter, der im Dunkeln isolierend ist, aber unter Belichtung leitend wird. Der Photoleiter kann aus einer oder mehreren Schichten bestehen. Im Fall einer einzigen Schicht ist mindestens eine photoleitfähige Substanz in mindestens einem Bindemittel dispergiert oder unmittelbar auf eine leitende Unterlage aufgedampft. Ein mehrschichtiger Photoleiter besteht bevorzugt aus mindestens einer photoleitfähigen Schicht, enthaltend eine oder mehrere photoleitfähige Substanzen, und mindestens einer ladungstransportierenden Schicht.

Die leitfähige Unterlage kann aus einer Metallplatte oder -folie bestehen, die roh oder z.B durch Aufrauhen vorbehandelt ist und z.B. aus Aluminium, Zink, Magnesium, Kupfer oder einer Legierung dieser Metalle besteht. Im Falle des Aluminiums kann die Vorbehandlung in einem Anodisieren bestehen. Als Unterlagen kommen auch aluminium-bedampfte Kunststoff-Folien sowie Polymerfilme mit metallisierter Oberfläche in Frage.

Der Photoleiter enthält als photoleitfähige Verbindungen mindestens eine Verbindung der Formel I und als ladungstransportierende Substanzen Verbindungen, wie z.B. Hydrazone, Oxadiazole, Oxazole oder Pyrazoline, sowie Arylamine, welche im Polymer-Bindemittel gelöst sind. Ein solcher Aufbau erlaubt nach vorgängiger statischer Aufladung und bildmässiger Belichtung die Erzeugung eines entsprechenden Musters aufgeladener und entladener Stellen (latentes Bild), welches nach bekannten Verfahren der Reprographie in ein sichtbares Abbild übergeführt werden kann.

Die Belichtung kann mit Licht im sichtbaren Wellenbereich erfolgen.

Zur Erhaltung des statischen Potentials an Stellen, die nicht belichtet werden, tragen die Verbindungen der Formel I dadurch bei, dass sie einen hohen Dunkelwiderstand aufweisen.

Besteht der Photoleiter aus einer einzigen Schicht, so enthält diese eine oder mehrere Verbindungen der Formel I zweckmässig in fein verteilter Form, gegebenenfalls zusammen mit ladungstransportierenden Substanzen, in einem organischen Bindemittel. Das Bindemittel ist zweckmässig filmbildend, isolierend und adhäsiv. Je nach Anwendung ist es löslich in organischen Lösungsmitteln oder in basischen Mischungen organischer Lösungsmittel, die gegebenenfalls Wasser enthalten. Besonders geeignet sind Bindemittel auf der Basis von Polykondensations- und Polyadditionsprodukten, wie Polyamiden, Polyurethanen, Polyestern, Epoxyharzen, Phenoxyharzen, Polyketonen, Polycarbonaten, Polyvinylketonen, Polystyrolen, Polyvinylcarbazolen, Polyacrylamiden, Polymethylmethacrylaten, Polyvinylbutyrat, Polyvinylchlorid, Polyvinylacetat sowie Copolymerisaten, wie z.B. Styrol-Maleinsäureanhydrid-Copolymeren, Styrol-Methacrylsäure-Methacrylsäureester-Copolymeren oder Vinylchlorid-Vinylacetat-Copolymeren.

Besteht der Photoleiter aus mehreren Schichten, so sind Doppelschichten von besonderem Interesse. Für diesen Fall wird auf die leitfähige Unterlage zuerst eine photoleitfähige und auf diese eine zweite ladungstransportierende Schicht aufgebracht. Das Aufbringen der Schichten kann auch in umgekehrter Reihenfolge durchgeführt werden. Eine der Schichten, vorzugsweise die ladungserzeugende Schicht, enthält mindestens eine Verbindung der Formel I. Diese kann in einem organischen Bindemittel gelöst oder fein verteilt sein. Die Applikation auf die leitfähige Unterlage erfolgt beispielsweise durch Auftragen einer Lösung beziehungsweise Dispersion der Bindemittel-Farbkörper-Mischung in einem organischen Lösungsmittel und anschliessendes Verdampfen des Lösungsmittels. Man kann die Verbindung der Formel I aber auch auf die leitfähige Unterlage aufdampfen.

Die zweite Schicht enthält eine oder mehrere ladungstransportierende Substanzen, vorzugsweise gelöst oder dispergiert in einem organischen Bindemittel. Als ladungstransportierende Substanzen kommen die verschiedensten aromatischen, vorzugsweise stickstoffhaltigen Verbindungen in Frage, wie Hydrazone oder aromatische Amine, die gegebenenfalls Alkylidenbrücken oder -reste enthalten. Beispielsweise handelt es sich um die in der Deutschen Offenlegungsschrift 34 47 685 auf den Seiten 57-65 sowie in der Publikation "Japan, Hardcopy '88, Post-International Symposium in Kansa; Recent Progress in Hardcopy Materials in Electrophotography", Seite 22 (Osaka, JP, 23.5.88) beschriebenen Substanzen.

Zur Verbesserung der physikalischen Eigenschaften der Schichten können sowohl die photoleitfähige als auch die ladungstransportierende Schicht noch Zusätze, wie Egalisiermittel, oberflächenaktive Mittel oder Weichmacher, enthalten.

Die nachstehenden Beispiele erläutern die Erfindung:
Beispiel 1: In einem 1 l Stahl-Autoklav werden unter Stickstoff 20 g 1,4-Diketo-3,6-bis-(4-bromphenyl)-pyrrolo[3,4-c]-pyrrol, 16,7 g Dimethylamin-Gas und 400 ml wasserfreien N-Methylpyrrolidon vorgelegt. Unter mässigem Rühren wird die Suspension in etwa 75 Minuten auf 180°C erhitzt und 10 Stunden bei dieser Temperatur gehalten. Der Druck beträgt während dieser Zeit ca. 1 bar. Anschliessend wird die Reaktionsmasse auf Raumtemperatur abgekühlt, der Autoklav wird geöffnet und die Suspension wird abgenutscht. Der Filterkuchen wird nacheinander mit Dimethylformamid, Methanol und warmem Wasser gründlich gewaschen, und das erhaltene Feuchtpigment wird bei ca. 80°C im Vakuumtrockenschrank getrocknet. Man erhält 11,6 g (69,3 % d.Th.) der Verbindung der Formel 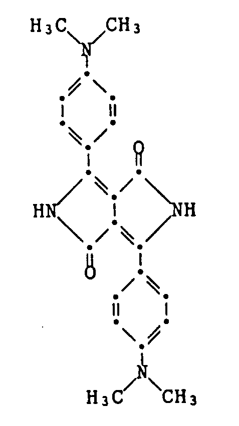
in Form eines blauvioletten Pulvers.

Beispiel 2: Man verfährt wie in Beispiel 1 beschrieben, setzt aber statt 16,7 g Dimethylamin-Gas 25,9 g wasserfreies Pyrrolidin ein und erhitzt auf 200°C anstatt nur auf 180°C. Der Druck beträgt ca. 2 bar. Man erhält 11,56 g (60,5 % d.Th.) der Verbindung der Formel 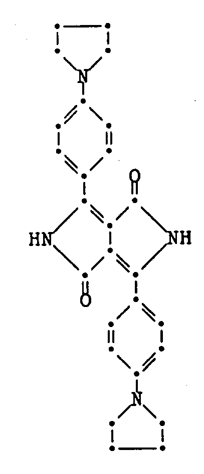
in Form eines blauvioletten Pulvers.

Beispiel 3: Man verfährt wie in Beispiel 1 beschriebene, setzt aber statt 16,7 g Dimethylamin-Gas 31 g wasserfreies Piperidin ein und erhizt 15 Stunden auf 180°C anstatt 10 Stunden. Der Druck beträgt ca. 1 bar. Man erhält 10,6 g (52,1 % d.Th.) der Verbindung der Formel 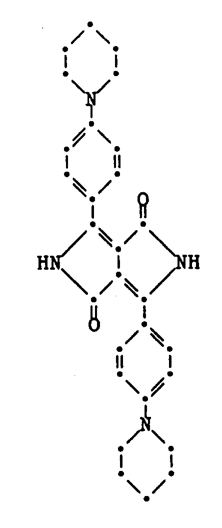
in Form eines violetten Pulvers.

Beispiel 4: Man verfährt wie in Beispiel 1 beschrieben, setzt aber statt 16,7 g Dimethylamin-Gas 31,7 g wasserfreies Morpholin ein und erhitzt 10 Stunden auf 220°C anstatt auf 180°C. Der Druck beträgt ca. 2 bar. Man erhält 10,2 g (49,7 % d.Th.) der Verbindung der Formel 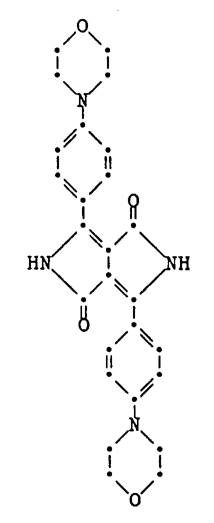
in Form eines violetten Pulvers.

Beispiel 5: In einen 1-Liter Stahl-Autoklav werden unter Stickstoff 25 g 1,4-Diketo-3,6-bis-(4-chlorphenyl)-pyrrolo[3,4-c]-pyrrol, 40,2 g wasserfreies Pyrrolidin und 500 ml wasserfreier N,N'-Dimethylethylenharnstoff vorgelegt. Unter mässigem Rühren wird die Suspension in etwa 2½ Stunden auf 200°C erhitzt und 10 Stunden bei dieser Temperatur gehalten. Der Druck beträgt während dieser Zeit ca. 2 bar. Anschliessend wird die Reaktionsmasse auf Raumtemperatur abgekühlt und wie in Beispiel 1 beschrieben aufgearbeitet. Man erhält 13,62 g (45,6 % d.Th.) der gleichen Verbindung, wie gemäss Beispiel 2.

Beispiel 6: In einem kleinen Druck-Reagenzglas von 20 ml Inhalt (mit Sicherheitsventil für max. 10 bar) werden zusammen 0,75 g 1,4-Diketo-3-(4-fluorphenyl)-6-phenylpyrrolo[3,4-c]pyrrol, 0,97 ml Piperidin und 11,3 ml wasserfreies Dimethylsulfoxid vorgelegt. Das Gefäss wird bei Raumtemperatur verschraubt und auf 160°C (Aussentemperatur) unter Rühren aufgeheizt. (Das Gefäss wird im Heizbad von ca. 160°C gedreht). Nach 23 Std. Ausrühren bei dieser Temperatur wird der Reaktor auf Raumtemperatur abgekühlt, geöffnet und die erhaltene Suspension filtriert. Der Niederschlag wird noch mit wenig Dimethylsulfoxid ausgespült und anschliessend mit Wasser gewaschen. Nach der Trocknung im Vakuumtrockenschrank bei 60°C erhält man 0,60 g (ca. 65 % d.Th.) einer Verbindung der Formel: 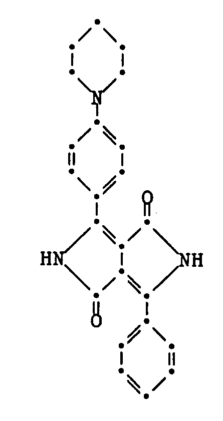
in Form eines rot-violetten Pulvers.

Beispiel 7: In einem 300 ml Stahl-Autoklav werden unter Stickstoff 5 g 1,4-Diketo-3,6-bis-(4-bromphenyl)-pyrrolo[3,4-c]-pyrrol, 7,81 g N-Methylbutylamin und 100 ml wasserfreies N-Methylpyrrolidon vorgelegt. Unter mässigem Rühren wird die Suspension in etwa 2 Stunden auf 200°C erhitzt und 24 Stunden bei dieser Temperatur gehalten. Der Druck beträgt während dieser Zeit 1 bis 2 bar. Anschliessend wird die Reaktionsmasse auf Raumtemperatur abgekühlt, der Autoklav wird geöffnet und die Suspension abgenutscht. Der Filterkuchen wird nacheinander mit N-Methylpyrrolidon und Methanol gründlich gewaschen, und das erhaltene Feuchtpigment wird bei ca. 80°C im Vakkumtrockenschrank getrocknet. Man erhält 1,0 g (20 % d.Th.) der Verbindung der Formel 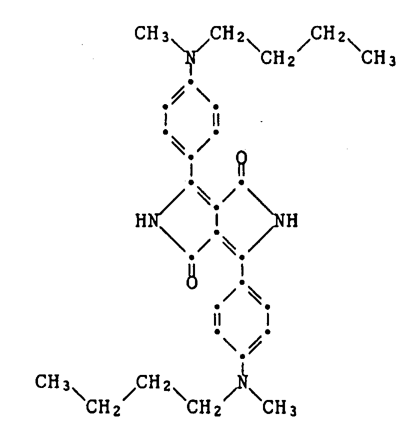
in Form eines blauvioletten Pulvers.

Beispiel 8: 0,3 g des Produktes von Beispiel 1 werden in einem Gemisch von 10 g Xylol und Ethylenglykolmonomethylether (2:1 in Volumen), enthaltend 1,0 g eines handelsüblichen Alkyd/Melaminharz (1:1 in Gewicht), aufgenommen. Die Suspension wird dann während 5 Stunden mit Glaskugeln gemahlen und anschliessend auf eine Aluminiumplatte mit einem Ziehstab aufgetragen (=ladungserzeugende Schicht). Diese Schicht wird bei 50°C während 3 Stunden getrocknet. Die Schichtdicke beträgt ca. 1 »m. Eine zweite Schicht, bestehend aus einem Gemisch von 0,6 g eines Hydrazons der Formel 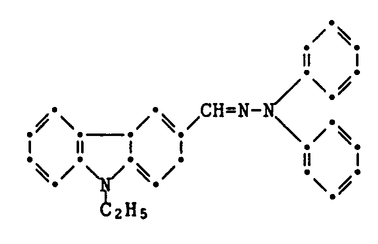
und 0,9 g des Polyacrylatlackes ®Lucite 41 in 11 g Methylethylketon, wird dann aufgetragen und bei 50°C während 15 Stunden getrocknet. Die Schichtdicke beträgt 1 bis 15 »m. Dieses Photorezeptor zeigt eine Empfindlichkeit (E ½) von 5 »J/cm², und die Aufladbarkeit beträgt 650 Volt.

Beispiel 9: Das Produkt von Beispiel 1 wird mit einer Geschwindigkeit von 5 Å/Sek. unter einem Vakuum von 10⁻⁶ mbar auf eine Aluminium-Unterlage aufgedampft. Die erhaltene Schichtdicke beträgt ca. 1000 Å. Eine zweite Schicht, bestehend aus einem Gemisch von 0,6 g eines Hydrazons der Formel 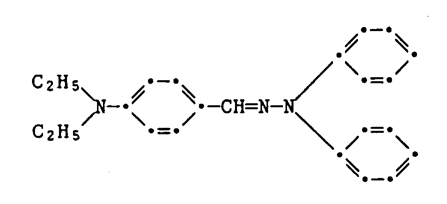
und 0,6 g Polycarbonat (®Makrolon, DuPont) in 10 g Tetrahydrofuran, wird anschliessend aufgetragen und bei 50°C während 6 Stunden getrocknet. Die Schichtdicke beträgt ca. 15 »m. Dieser Photorezeptor zeigt eine Lichtempfindlichkeit (E ½) von 8 »J/cm².

## Patentansprüche

1. Verfahren zur Herstellung von Verbindungen der Formel 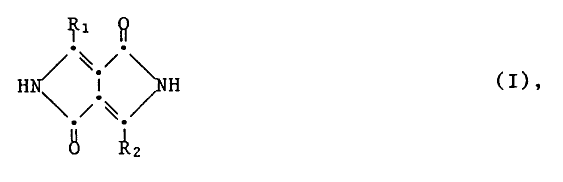 worin R₁ eine Gruppe 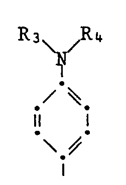 und R₂ eine Gruppe 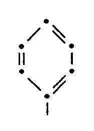 oder R₁ bedeuten, wobei R₃ und R₄ gleich sind und C₁-C₁₂-Alkyl, 2-Hydroxyethyl, 2-Mercaptoethyl, Cyclohexyl, Benzyl oder Phenylethyl bedeuten oder zusammen mit dem N-Atom, an das sie gebunden sind, Pyrrolidinyl, Piperidyl, Morpholinyl oder Thiomorpholinyl bilden,
durch Umsetzung eines Pyrrolopyrrols der Formel 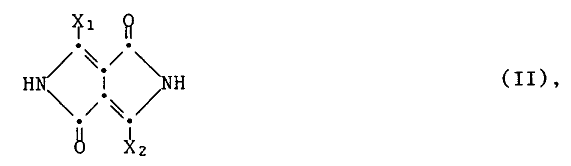 worin X₁ eine Gruppe 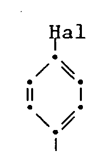 und X₂ eine Gruppe 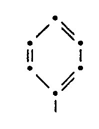 oder X₁ bedeuten, Hal Chlor oder Brom ist, mit einem sekundären Amin der Formel 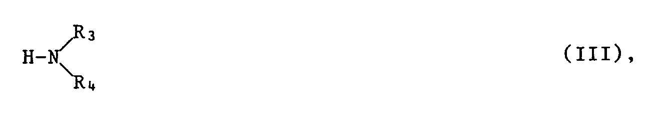 worin R₃ und R₄ die oben bereits angegebene Bedeutung haben, im Molverhältnis 1:1 oder, wenn X₂ die gleiche Bedeutung wie X₁ hat, im Molverhältnis 1:2, in Gegenwart eines wasserfreien, dipolaren, aprotischen Lösungsmittels ausgewählt aus der Gruppe bestehend aus Carbonsäureamiden, Lactamen, Harnstoffderivaten, Sulfonen und Nitrobenzol und eines als Base wirkenden, 0,1 bis 15 Mol betragenden Ueberschusses an Amin der Formel III bei Temperaturen zwischen 100 und 220°C und einem Druck zwischen 1 und 3 bar.

2. Verfahren gemäss Anspruch 1, dadurch gekennzeichnet, dass in den Formeln I, II und III R₁ und R₂ und demnach auch X₁ und X₂ gleich sind, R₃ und R₄ C₁-C₄-Alkyl, 2-Hydroxyethyl, 2-Mercaptoethyl bedeuten oder zusammen mit dem N-Atom, an das sie gebunden sind, Pyrrolidinyl, Piperidyl, Morpholinyl oder Thiomorpholinyl bilden, Hal Chlor oder Brom bedeuten, und dass die Umsetzung bei Temperaturen zwischen 150 und 200°C durchgeführt wird.

3. Verfahren gemäss Anspruch 2 zur Herstellung von Verbindungen der Formel I, worin R₁ und R₂ gleich sind und eine der Gruppen 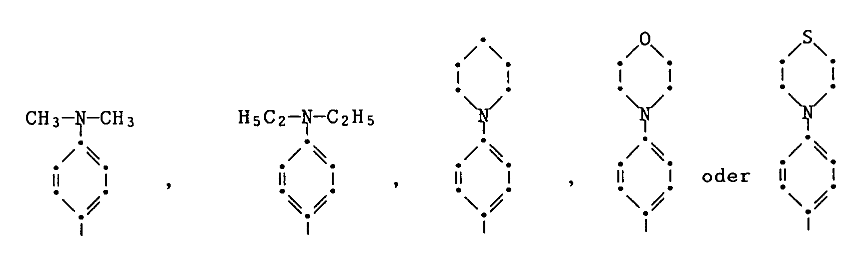 bedeuten.

4. Verfahren gemäss Anspruch 1, dadurch gekennzeichnet, dass man als Lösungsmittel ein dipolares, aprotisches Lösungsmittel ausgewählt aus der Gruppe bestehend aus Dimethylformamid, Dimethylacetamid, N-Methylpyrrolidon, N,N'-Dimethylethylenharnstoff und N,N'-Dimethylpropylenharnstoff verwendet.

## Claims

1. A process for the preparation of a compound of formula 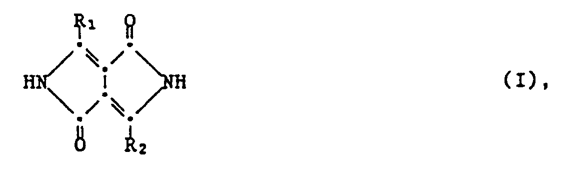 in which R₁ is a group 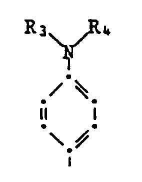 and R₂ is a group 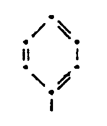 or has the meaning of R₁, in which formulae R₃ and R₄ are identical and are C₁-C₁₂alkyl, 2-hydroxyethyl, 2-mercaptoethyl, cyclohexyl, benzyl or phenylethyl, or, together with the N atom to which they are attached, form pyrrolidinyl, piperidyl, morpholinyl or thiomorpholinyl,
which process comprises reacting a pyrrolopyrrole of formula 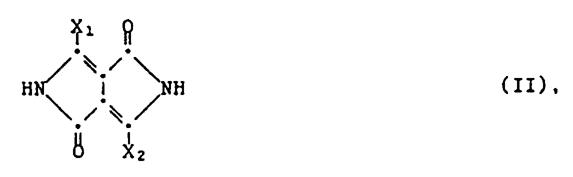 in which X₁ is a group 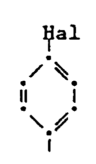 and X₂ is a group 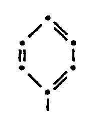 or has the same meaning as X₁, and Hal is chlorine or bromine, with a secondary amine of formula 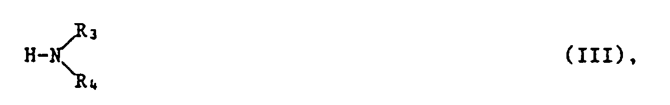 in which R₃ and R₄ are as defined above, in the molar ratio 1:1 or, if X₂ has the same meaning as X₁, in the molar ratio 1:2, in the presence of an anhydrous dipolar aprotic solvent selected from the group consisting of carboxamides, lactams, urea derivatives, sulfones and nitrobenzene and of a 0.1 to 15 molar excess of an amine of formula III acting as base, in the temperature range from 100 to 220°C and under a pressure from 1 to 3 bar.

2. A process according to claim 1, wherein R₁ and R₂ in formulae I, II and III and hence X₁ and X₂ are identical, R₃ and R₄ are C₁-C₄alkyl, 2-hydroxyethyl, 2-mercaptoethyl, or, together with the N atom to which they are attached, form pyrrolidinyl, piperidyl, morpholinyl or thiomorpholinyl, and Hal is chlorine or bromine, and wherein the reaction is carried out in the temperature range from 150 to 200°C.

3. A process according to claim 2 for the preparation of a compound of formula I, in which R₁ and R₂ are identical and are each a group selected from

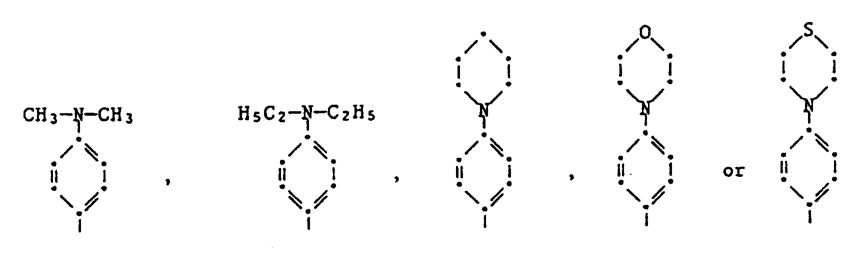

4. A process according to claim 1, wherein the solvent is a dipolar aprotic solvent selected from the group consisting of dimethylformamide, dimethylacetamide, N-methylpyrrolidone, N,N'-dimethylethyleneurea and N,N'-dimethylpropyleneurea.

## Revendications

1. Procédé pour la préparation des composés de formule : 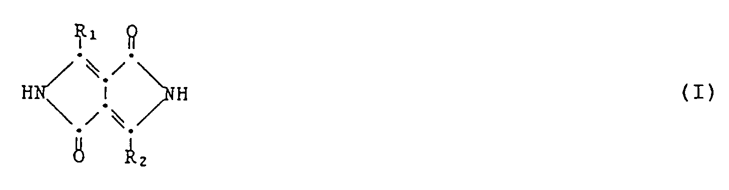 dans laquelle R₁ représente un groupe 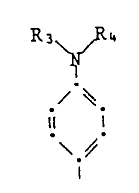 et R₂ un groupe 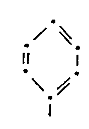 ou R₁, R₃ et R₄ étant identiques et signifiant un alkyle en C₁-C₁₂, le 2-hydroxyéthyle, le 2-mercapto-éthyle, le cyclohexyle, le benzyle ou le phényléthyle, ou ensemble avec l'atome de N auquel ils sont liés, forment un pyrrolidinyle, un pipéridyle, un morpholinyle ou thiomorpholinyle,
par réaction d'un pyrrolopyrrole de formule 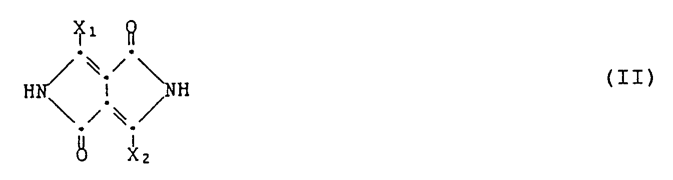 dans laquelle X₁ représente un groupe 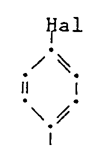 et X₂ un groupe 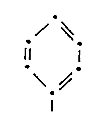 ou X₁, Hal est le chlore ou le brome, avec une amine secondaire de formule 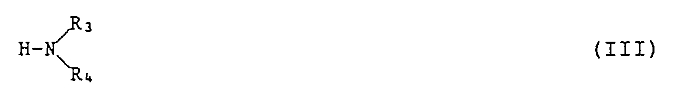 dans laquelle R₃ et R₄ ont les significations déjà données ci-dessus, dans un rapport molaire 1:1 ou, lorsque X₂ a la même signification que X₁, dans un rapport molaire 1:2, en présence d'un solvant aprotique dipolaire exempt d'eau pris dans le groupe comprenant les amides d'acides carboxyliques, les lactames, les dérivés de l'urée, les sulfones et le nitrobenzène et un excédent d'amine de formule III en quantité de 0,1 à 15 moles, agissant en tant que base, à des températures comprises entre 100 et 220 °C et à des pressions comprises entre 1 et 3 bar.

2. Procédé selon la revendication 1, caractérisé en ce que dans les formules I, II et III R₁ et R₂ et, par conséquent aussi X₁ et X₂, sont identiques, R₃ et R₄ représentent un alkyle en C₁-C₄, le 2-hydroxy-éthyle, le 2-mercapto-éthyle ou, ensemble avec l'atome de N auquel ils sont liés, forment un pyrrolidinyle, un pipéridyle, un morpholinyle ou thiomorpholinyle, Hal signifie le chlore ou le brome, et en ce que la réaction est effectuée à des températures comprises entre 150 et 200 °C.

3. Procédé selon la revendication 2 pour la préparation de composés de formule I, dans lesquels R₁ et R₂ sont identiques et représentent un des groupes

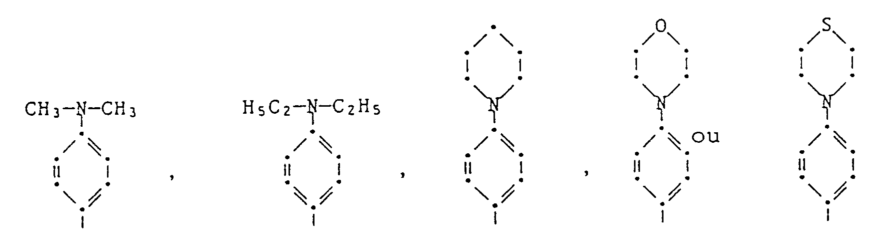

4. Procédé selon la revendication 1, caractérisé en ce qu'on utilise en tant que solvant un solvant aprotique dipolaire pris dans le groupe comportant le diméthylformamide, le diméthylacétamide, la N-méthylpyrrolidone, la N,N'-diméthyléthylène-urée et la N,N'-diméthylpropylène-urée.
